# EUROPÄISCHE PATENTSCHRIFT

(11) **EP 1 446 220 B1**
(45) Veröffentlichungstag und Bekanntmachung des Hinweises auf die Patenterteilung: **20.07.2005**
(21) Anmeldenummer: 02779548.3
(22) Anmeldetag: 11.11.2002
(51) Int. Cl.: B01F 17/00, A61K 9/00, A61K 7/00

(54) **EMULGATOR-GEMISCH**
EMULSIFIER MIXTURE
MELANGE EMULSIFIANT

(30) Priorität: 19.11.2001 DE 10156285
(43) Veröffentlichungstag der Anmeldung: 18.08.2004
(73) Patentinhaber: Cognis IP Management GmbH, 40589 Düsseldorf (DE)
(72) Erfinder: SCHMID, Karl, Heinz, 40822 Mettmann (DE); LEMKE, Ute, 41470 Neuss (DE); MAMPE, Dirk, 40476 Düsseldorf (DE); HILL, Karlheinz, 40699 Erkrath (DE); HÜBNER, Norbert, 40764 Langenfeld (DE)
(86) Internationale Anmeldenummer: PCT/EP2002/012560
(87) Internationale Veröffentlichungsnummer: WO 2003/043725

(56) Entgegenhaltungen:
- EP-A- 0 895 805
- WO-A-00/56438
- WO-A-92/06778
- WO-A-95/13863
- WO-A-98/22207
- DE-A- 19 542 572

## Beschreibung

### Gebiet der Erfindung

Die Erfindung betrifft Emulgator-Compounds mit einem definierten Gehalt an verzweigten, ungesättigten Fettalkoholen und Isoalkenyl(oligo)glykosiden sowie die Verwendung des Emulgator-Compounds zur Herstellung von kosmetischen und/oder pharmazeutischen Zubereitungen und als Rückfettungsmittel in kosmetischen und pharmazeutischen Zubereitungen.

### Stand der Technik

Im Bereich kosmetischer Emulsionen für die Haut- und Haarpflege werden vom Verbraucher eine Vielzahl von Anforderungen gestellt: abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel ein oder mehrere oberflächenaktive Substanzen, insbesondere auf Basis von anionischen oder amphoteren Tensiden. Da die alleinige Verwendung von Tensiden Haut und Haare zu sehr austrocknen würde, ist es im allgemeinen üblich, solchen Mitteln auch rückfettende Substanzen zuzusetzen. Es liegt dabei auf der Hand, daß diese Stoffe nicht nur eine hinreichende rückfettende Wirkung, sondem den Erfordernissen des Marktes folgend gleichzeitig auch eine optimale dermatologische Verträglichkeit aufweisen müssen.

Aus der Deutschen Patentschrift **DE-C2 41 39 935** sind flüssige Körperreinigungsmittel auf wäßriger Basis bekannt, die 5 bis 35 Gew.-% anionische Tenside, 2,5 bis 15 Gew.-% Alkylpolyglucoside und 0,5 bis 15 Gew.-% gesättigte Fettsäuremonoglyceride mit 8 bis 18 Kohlenstoffatomen im Fettacylrest aufweisen. Die in diesem Dokument vorgeschlagenen Monoglyceride weisen jedoch auch in Abmischung mit Glucosiden eine nicht ausreichende dermatologische Verträglichkeit auf. Zudem sind die Mischungen in Abwesenheit von Wasser in der Regel fest und lassen sich daher nicht kalt verarbeiten.

Gemäß der Lehre der Deutschen Patentanmeldung **DE-A 27 01 266** sind Schaumbadzusammensetzungen bekannt, die 1 bis 50 Gew.-% Fettsäuremonoglyceride und 5 bis 50 Gew.-% Alkylsulfate, Alkylethersulfate und/oder Ethercarbonsäuresalze enthalten können. Diese Mischungen weisen jedoch keine vorteilhaften rückfettenden Eigenschaften auf.

Gegenstand der Europäischen Patentschrift **EP-B1 0 554 292** sind O/W-Emulsionen, die Ölkörper, Alkylpolyglucoside, Fettsäurepartialglyceride und gegebenenfalls Fettalkohole enthalten. Auch diese Mischungen sind hinsichtlich ihrer rückfettenden Wirkung, ihrer Hautverträglichkeit und Konsistenz in wasserfreiem Zustand nicht vollkommen zufriedenstellend.

Aus der Europäischen Patentanmeldung **EP-A1 0 538 762** sind schließlich Haarbehandlungsmittel bekannt, die kationische Tenside, Alkylpolyglucoside und Ölkörper, beispielsweise auch Fettsäuremonoglyceride enthalten, die dem Harr Weichheit und gute Elastizität verleihen sollen.

Aus der Europäischen Patentschrift **EP-B1 0 553 241** ist die Verwendung von autoemulgierbaren Mischungen aus 10 - 40 Gew.-% Alkyloligoglucosiden, 60 - 90 Gew.-% Fettalkoholen und gegebenenfalls Polyglucose zur Herstellung von Emulsionen bekannt. Gemäß der Lehre der internationalen Patentanmeldung WO 97/18033 läßt sich ein Emulgatorkonzentrat aus 43 - 90 Gew.-% Alkyl- und/oder Alkenyloligoglykoside und 10 bis 57 Gew.-% Fettalkoholen zur Herstellung kosmetischer Emulgatoren einsetzen. Diese Zusammensetzungen weisen keine vorteilhaften rückfettenden Eigenschaften auf und sind ebenfalls fest, so daß sie sich nicht kalt verarbeitbar sind.

Aufgabe der vorliegenden Erfindung war es, bei Raumtemperatur flüssige Emulgator-Konzentrate (Compounds) zur Herstellung kosmetischer Produkte zur Verfügung zu stellen, die neben guten oberflächenaktiven Eigenschaften auch gute rückfettende sowie verdickende Eigenschaften aufweisen. Die Emulgator-Konzentrate sollten lagerstabil und für kosmetische Applikationen farblich geeignet sein und sich besonders leicht in Kaltprozessen in kosmetische Zubereitungen einarbeiten lassen.

### Beschreibung der Erfindung

Es wurde gefunden, daß sich Emulgator-Konzentrate mit einem Gehalt an verzweigten, ungesättigten Fettalkoholen und Isoalkenyl(oligo)glykosiden bei Raumtemperatur flüssig sind und sich besonders leicht in Kaltprozessen in Emulsionen einarbeiten lassen, eine gute dermatologische Verträglichkeit bei optimalen rückfettende Eigenschaften aufweisen und sehr lagerstabil sind. Ein weiterer Vorteil besteht darin, daß die Mischungen in tensidischen Systemen eine Viskosität aufbauen.

Gegenstand der Erfindung sind daher Emulgator-Konzentrate enthaltend:
(a) 0,5 bis 90 Gew.-% wenigstens eines iso-Alkenyloligoglykosids,
(b) 0,5 bis 90 Gew.% wenigstens eines iso-Alkenyl-Fettalkohols und
(c) 0 - 10 Gew.-% Wasser

Vorzugsweise sind die Iso-Alkenylreste des Oligoglykosids und des Fettalkohols identisch.

Ein weiterer Gegenstand der Erfindung ist die Verwendung der erfindungsgemäßen Emulgator-Konzentrate zur Herstellung kosmetischer oder pharmazeutischer Zubereitungen und als Rückfettungsmittel in kosmetischen oder pharmazeutischen Zubereitungen.

Die erfindungsgemäßen Emulgator-Konzentrate haben den Vorteil, daß sie aufgrund ihrer langen Alkylkette eine hohe Emulgierwirkung haben und daß sie trotz dieser langen Alkylkette bis zu einer Temperatur von 5 °C flüssig und somit kaltverarbeitbar sind. Im Vergleich zu linearen ungesättigten Alkohole sind die erfindungsgemäßen Emulgator-Konzentrate außerdem oxidationsstabiler und liefern daher bessere farbliche Qualitäten. Dies ist für den Einsatz in der Kosmetik besonders wichtig, da hier zersetzungsbedingte Verfärbungen völlig unakzeptabel sind und die so entstehenden Nebenprodukte auch zu Hautreizungen führen können. Auch die rückfettenden Eigenschaften sind besser als bei Mischungen auf Basis gesättigter und/oder unverzweigter Alkohole.

Die Konzentrate können herstellungsbedingt noch geringe Mengen an Osen, Polyosen, Wasser und anderen reaktionsbedingten Nebenprodukten enhalten, die jedoch üblicherweise 15% nicht übersteigen. Der Wassergehalt liegt bei höchstens 10 Gew.-%, vorzugsweise unter 5 Gew.-% bezogen auf das Emulgatorkonzentrat. Insbesondere bevorzugt sind wasserfreie Emulgatorkonzentrate. Unter wasserfrei im Sinne der Erfindung sind Emulgatorkonzentrate zu verstehen, die lediglich rohstoffbedingte Restwassermengen enthalten, denen aber kein zusätzliches Wasser zugesetzt wurde.

Je nach Applikationszweck überwiegt der Gehalt an Iso-Alkenyloligoglykosid oder an Iso-Alkenyl-Fettalkohol. Stehen rückfettende Eigenschaften im Vordergrund, so wird der Anteil an Iso-Alkenyl-Fettalkohol im Konzentrat höher gewählt. Für emulgierende Eigenschaften wird dagegen der Gehalt an iso-Alkenyloligoglykosid höher gewählt werden.

Vorzugsweise enthält das Konzentrat (a) 15 bis 80 Gew.-% wenigstens eines iso-Alkenyloligoglykosids, (b) 20 bis 85 Gew.-% wenigstens eines iso-Alkenyl-Fettalkohols und (c) 0 - 10 Gew.-% Wasser. Eine bevorzugte Ausführungsform des Konzentrates enthält (a) 20 bis 60 Gew.-% wenigstens eines iso-Alkenyloligoglucosids, (b) 40 bis 80 Gew.-% wenigstens eines iso-Alkenyl-Fettalkohols und (c) 0 - 10 Gew.-% Wasser.

### Iso-Alkenyl-Fettalkohole

Bei den Fettalkoholen handelt es sich um verzweigte, ungesättigte Fettalkohole, die oberhalb von 10 °C eine flüssige Konsistenz aufweisen. Vorzugsweise handelt es sich um C₁₀-C₅₄-Iso-Alkenylalkohole und insbesondere C₁₀-C₃₀-Iso-Alkenylalkohole und ganz besonders bevorzugt um C₁₆-C₂₂-Iso-Alkenylalkohole, die natürlichen oder synthetischen Ursprungs sein können. In der Alkenylkette ist wenigstens eine Verzweigungsstelle vorhanden, die synthesebedingt auch statistisch verteilt sein kann. Vorzugsweise sind nicht mehr als drei Doppelbindungen in der Alkylkette vorhanden, die vorzugsweise nicht konjugiert sind. Als Fettalkohol-Komponente ist erfindungsgemäß Iso-Oleylalkohol bevorzugt geeignet, insbesondere methylverzweigter Oleylalkohol, also ein Methylheptadecen. Dieser Iso-Oleylalkohol wird erhalten durch Hydrierung der sog. Monomerfettsäure bzw. des Monomerfettsäuremethylesters, die als Nebenprodukt bei der Dimerisierung von Ölsäure/Linolsäuregemischen anfällt. Erfindungsgemäß können auch beliebige Gemische verzweigter, ungesättigter Fettalkohole als iso-Alkenyl-Komponente eingesetzt werden, wie z.B. Iso-Erucaalkohol oder Iso-Brassidylalkohol bzw. deren Gemische, oder auch Abmischungen dieser beiden C₂₂-Alkohle mit Iso-Oleylalkohol.

### Iso-Alkenyloligoglykoside

Das Emulgator-Konzentrat enthält vorzugsweise iso-Alkenyloligoglykoside der Formel (I)

R¹O-[G]ₚ (I)

in der R¹ für einen iso-Alkenylrest mit 10 bis 54 Kohlenstoffatomen, G für einen Zuckerrest mit 5 oder 6 Kohlenstoffatomen und p für Zahlen von 1 bis 10 steht. Vorzugsweise steht R¹ für einen Iso-Alkenylrest mit 10 bis 30 Kohlenstoffatomen und ganz besonders bevorzugt 16 bis 22 Kohlenstoffatomen. In der Alkenylkette ist wenigstens eine Verzweigungsstelle vorhanden, die synthesebedingt auch statistisch verteilt sein kann . Vorzugsweise sind nicht mehr als drei Doppelbindungen in der Alkenylkette vorhanden, die vorzugsweise nicht konjugiert sind.

Diese Zuckertenside können nach den einschlägigen Verfahren der präparativen organischen Chemie erhalten werden. Stellvertretend für das umfangreiche Schrifttum sei hier auf die Schriften **EP 0 301 298 A1** und **WO 90/03977** verwiesen.

Die iso-Alkenyloligoglykoside können sich von Aldosen bzw. Ketosen mit 5 oder 6 Kohlenstoffatomen ableiten, beispielsweise Glucose, Dextrose, Sacccharose, Fructose, Galactose, Maltose, Maltotriose, Lactose, Cellobiose, Mannose, Ribose, Talose, Allose, Altrose, Xylose, Lyxose, Gulose, Idose, Arabinose, ferner Dextrane, Levoglucosane, und Cellulose. Erfindungsgemäß bevorzugt ist Glucose, die bevorzugten iso-Alkenyloligoglykoside sind-somit iso-Alkenyloligoglucoside.

Die Indexzahl p in der allgemeinen Formel (I) gibt den Oligomerisierungsgrad (DP) an, d. h. die Verteilung von Mono- und Oligoglykosiden, und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes iso-Alkenyloligoglykosid eine analytisch ermittelte rechnerische Größe, die meistens eine gebrochene Zahl darstellt. Vorzugsweise werden iso-Alkenyloligoglykoside mit einem mittleren Oligomerisierungsgrad p von 1,1 bis 3,0 eingesetzt. Aus anwendungstechnischer Sicht sind solche iso-Alkenyloligoglykoside bevorzugt, deren Oligomerisierungsgrad kleiner als 1,7 ist und insbesondere zwischen 1,2 und 1,4 liegt.

Eine bevorzugte Ausführungsform des Konzentrates enthält (a) 0,5 bis 90 Gew.-% wenigstens eines iso-Oleyl-Oligoglucosids, (b) 0,5 bis 90 Gew.-% wenigstens eines iso-Oleylalkohols und (c) 0 - 10 Gew.-% Wasser. Besonders bevorzugt sind Konzentrate mit (a) 15 bis 80 Gew.-% wenigstens eines iso-Oleyl-Oligoglucosids, (b) 20 bis 85 Gew.-% wenigstens eines iso-Oleylalkohols und (c) 0 - 10 Gew.-% Wasser und insbesondere Konzentrate mit (a) 20 bis 60 Gew.-% wenigstens eines iso-Oleyl-Oligoglucosids, (b) 40 bis 80 Gew.-% wenigstens eines iso-Oleylalkohols und (c) 0 - 10 Gew.-% Wasser. Derartige Konzentrate sind selbst bei 5 °C flüssig, weisen eine besonders vorteilhafte Kombination aus rückfettenden und emulgierenden Eigenschaften auf, bei gleichzeitiger leichter Einarbeitbarkeit in Kaltprozessen zur Herstellung von Cremes, Lotionen und Shampoos.

### Herstellverfahren

Ein weiterer Gegenstand der Erfindung ist ein Verfahren zur Herstellung der erfindungsgemäßen Emulgator-Konzentrate, bei dem man einen Zucker (Ose) und überschüssigen iso-Alkenyl-Fettalkohol einer sauren Acetalisierung unterwirft
(a) wobei das Edukt-Verhältnis von Zucker zu iso-Alkenyl-Fettalkohol so eingestellt wird, daß hieraus nach der Synthese direkt das gewünschte Verhältnis von iso-Alkenyloligoglykosid zu iso-Alkenyl-Fettalkohol resultiert oder in dem resultierenden Gemisch aus iso-Alkenyloligoglykosiden und überschüssigem iso-Alkenyl-Fettalkohol
(b) der iso-Alkenyl-Fettalkoholanteil im Gemisch entweder durch Abdestillieren des Fettalkohols oder durch Zugabe von zusätzlichem iso-Alkenyloligoglykosid-Anteil auf den gewünschten Gehalt reduziert wird oder
(c) der iso-Alkenyl-Fettalkoholanteil im Gemisch durch nachträgliche Zugabe von iso-Alkenyl-Fettalkohol auf den gewünschten Gehalt erhöht.

Diese Verfahrensweise erlaubt es, kosmetisch akzeptable und dermatologisch besonders verträgliche Produkte von sehr guter farblicher und geruchlicher Qualität zu erhalten, da die Produkte unter Säurekatalyse weniger polymersieren als die ungesättigten, unverzweigten Analoga und wesentlich oxidationsstabiler sind.

Vorzugsweise geht man jedoch von technischen iso-Alkenyloligoglykosid/iso-Alkenyl-Fettalkoholgemischen aus, wie sie bei der Herstellung von iso-Alkenyloligoglykosiden durch säurekatalysierte Acetalisierung von Osen mit den entsprechenden Fettalkoholen im Überschuß anfallen. Diese Mischungen enthalten üblicherweise etwa 60 bis 85 Gew.-% iso-Alkenyl-Fettalkohol und 15 bis 40 Gew.-% der iso-Alkenyloligoglykoside.

Zur Einstellung eines höheren iso-Alkenyloligoglycosid-Anteils kann man grundsätzlich nach zwei Methoden verfahren: Entweder wird der iso-Alkenyl-Fettalkoholanteil durch eine anschließende Behandlung im Dünnschicht- oder Fallfilmverdampfer destillativ bis auf das gewünschte Maß abgereichert oder aber man stockt den iso-Alkenyloligoglycosid-Anteil durch vorzugsweise wasserfreie iso-Alkenyloligoglycoside auf, die man ihrerseits aus wäßrigen Pasten durch Heißdampftrocknung oder durch Entwässerung im Flash-Dryer herstellen kann. Konzentrate mit einem höheren iso-Alkenyloligoglycosid-Anteil zeigen gute rückfettende und gleichzeitig gute emulgierende Eigenschaften.

Zur Einstellung eines höheren iso-Alkenyl-Fettalkohol-Anteils wird nachträglich iso-Alkenyl-Fettalkohol zugegeben. Bei Emulgator-Konzentraten mit einem höheren Fettalkohol-Anteil stehen die rückfettenden Eigenschaften im Vordergrund.

In einer bevorzugten Ausführungsform der Erfindung werden Glucose und iso-Oleylalkohol in an sich bekannter Weise acetalisiert und überschüssiger iso-Oleylalkohol zum Teil abgetrennt, so daß Produkte erhalten werden, die mehr als 30 Gew.-% iso-Alkenylglucosid enthalten. Falls erforderlich, kann gebildete Polyglucose mit Hilfe von Membranen abgetrennt werden.

Ein weiterhin bevorzugtes Verfahren zur Herstellung der erfindungsgemäßen Emulgator-Konzentrate besteht darin, daß man z. B. Glucose oder Stärkesirup zunächst mit einem kurzkettigen Alkohol, beispielsweise Butanol oder einem Vorlauffettalkohol, zu einem Niedrigalkylglucosid umsetzt und dieses anschließend mit dem gewünschten langkettigen iso-Alkenyl-Fettalkohol, vorzugsweise iso-Oleylalkohol, umacetalisiert. Die weitere Aufbereitung kann dann wie oben geschildert erfolgen.

### Hydrophile Wachse

In einer bevorzugten Ausführungsform der Erfindung enthalten die Emulgator-Konzentrate zusätzlich ein konsistenzgebendes hydrophiles Wachs. Hierbei handelt es sich um bei Raumtemperatur feste Stoffe, die über freie Hydroxylgruppen verfügen. Typische Beispiele sind Fettsäurepartialglyceride, d.h. technische Mono- und/oder Diester des Glycerins mit Fettsäuren mit 12 bis 18 Kohlenstoffatomen wie etwa Glycerinmono/dilaurat, -palmitat, -oleat oder -stearat. Weiterhin kommen auch Fettalkohole wie etwa technische Fettalkohole mit 12 bis 22 Kohlenstoffatomen, namentlich Cetylalkohol, Stearylalkohol oder Cetearylalkohol in Betracht. Die hydrophilen Wachse sind nur in solchen Mengen enthalten, daß die Konsistenz des Gemisches dennoch flüssig bleibt. Derartige Optimierungen gehören zu Routine-Optimierungen des Fachmanns.

Vorteilhafte Mischungen aus den Emulgatorkomponenten (a), (b), (c) und hydrophilen Wachsen (c) enthalten beispielsweise:
(a) 10 bis 65 Gew.-% iso-Alkenyloligoglykoside,
(b) 10 bis 65 Gew.-% iso-Alkenyl-Fettalkohole,
(c) 0 - 10 Gew.-% Wasser und
(c) 0,1 bis 30 Gew.-% hydrophile Wachse, wie z.B. Fettsäurepartialglyceride.

Der Zusatz der hydrophilen Wachse erlaubt es, die rückfettenden Eigenschaften weiter zu verbessern und den erfindungsgemäßen Konzentraten auch stärker konsistenzgebende Eigenschaften zu verleihen, bei gleichzeitiger leichter Einarbeitbarkeit und Lagerstabilität der Konzentrate.

### Kosmetische/pharmazeutische Zubereitungen

Die erfindungsgemäßen Emulgatoren erlauben die Herstellung stabiler Emulsionen und Tensid-Formulierungen in Kaltprozessen. Im Gegensatz zu Emulgator-Konzentraten des Standes der Technik auf Basis gesättigter und unverzweigter Fettalkohole, zeigen sie bei vergleichbarem Emulgiervermögen ein weitaus besseres Rückfettungsvermögen, das für einen Großteil kosmetischer Applikationsformen relevant ist. Die erfindungsgemäßen Konzentrate zeigen auch ein besseres Emulgiervermögen, d.h. daß geringere Einsatzmengen des Konzentrates nötig sind, und die resultierenden Zusammensetzungen daher hautverträglicher sind. Ein weiterer Gegenstand der Erfindung sind daher kosmetische oder pharmazeutische Zubereitungen enthaltend 0,1 bis 30 Gew.-% der Emulgatorkonzentrate. Vorzugsweise handelt es sich hierbei um tensidische Formulierungen, bei denen gute Schaumwirkung in Kombination mit guten rückfettenden Eigenschaften und hoher dermatologischer Verträglichkeit gefragt sind, beispielsweise um Duschgele und -öle, Schaumbäder, Haarshampoos, Pflegespülungen, etc. Die erfindungsgemäßen Emulgator-Konzentrate können aber auch zur Formulierung von Körperpflegeprodukten eingesetzt werden, wie z. B. Cremes, Lotionen und Milchen, Produkten zur Eliminierung des Körpergeruchs u.ä. Je nach Applikationszweck enthalten die kosmetischen Formulierungen eine Reihe weiterer Hilfs- und Zusatzstoffe, wie beispielsweise Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe etc., die nachstehend exemplarisch aufgelistet sind.

### Tenside

Als oberflächenaktive Stoffe können anionische, nichtionische, kationische und/oder amphotere bzw. zwitterionische Tenside enthalten sein. Eine bevorzugte Ausführungsform der erfindungsgemäßen kosmetischen Zubereitung enthält zusätzlich wenigstens ein Tensid, vorzugsweise ein anionisches Tensid. In tensidhaltigen kosmetischen Zubereitungen, wie beispielsweise Duschgele, Schaumbäder, Shampoos, etc. ist die rückfettende Wirkung der erfindungsgemäßen Emulgator-Kombination besonders vorteilhaft. Der Anteil der zusätzlichen Tenside liegt üblicherweise bei etwa 1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Fettalkoholethercarboxylate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Ein besonders bevorzugtes anionisches Tensid ist Fettalkoholethersulfat.

### Ölkörper

Körperpflegemittel, wie Cremes, Lotionen und Milchen, die das erfindungsgemäße Emulgator-Konzentrat enthalten, sind ölkörperhaltig. Die Ölkörper sind üblicherweise in einer Menge von 1 - 50 Gew.-%, vorzugsweise 2 - 25 Gew.-% und inbesondere 2 - 15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl-Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als zusätzliche Emulgatoren kommen beispielsweise nichtionogene Tenside aus mindestens einer der folgenden Gruppen in Frage:
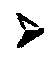 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
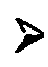 Alkyloligoglykoside mit 8 bis 22 Kohlenstoffatomen im Alkylrest und deren ethoxylierte Analoga;
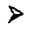 Anlagerungsprodukte von 1 bis 15 Mol Ethylenoxid an Ricinusöl und/oder gehärtetes Ricinusöl;
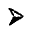 Anlagerungsprodukte von 15 bis 60 Mol Ethylenoxid an Ricinusöl und/oder gehärtetes Ricinusöl;
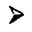 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
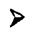 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
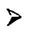 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
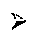 Mono-, Di- und Trialkylphosphate sowie Mono-, Di- und/oder Tri-PEG-alkylphosphate und deren Salze;
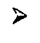 Wollwachsalkohole;
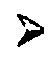 Polysiloxan-Polyalkyl-Polyether-Copolymere bzw. entsprechende Derivate;
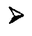 Block-Copolymere z.B. Polyethylenglycol-30 Dipolyhydroxystearate;
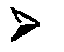 Polymeremulgatoren, z.B. Pemulen-Typen (TR-1,TR-2) von Goodrich;
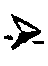 Polyalkylenglycole sowie
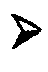 Glycerincarbonat.

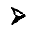 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

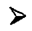 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

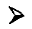 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

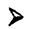 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

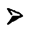 Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als- Emulgatoren in Betracht, wobei solche vom Typ der Esterquats, vorzugsweise methylquaternierte Difettsäuretriethanolaminester-Salze, besonders bevorzugt sind.

### Fette und Wachse

Fette und Wachse werden den Körperpflegeprodukten zugesetzt. Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B.

Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren-Mischungen.

### Konsistenzgener und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind; ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
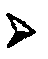 3-Benzylidencampher bzw. 3-Benzylidennorcampher und dessen Derivate, z.B. 3-(4-Methylbenzyliden)campher;
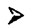 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
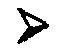 Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
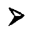 Ester der Salicylsäure, vorzugsweise Salicylsäure-2-ethylhexylester, Salicylsäure-4-iso-propylbenzylester, Salicylsäurehomomenthylester;
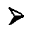 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
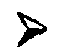 Ester der Benzalmalonsäure, vorzugsweise 4-Methoxybenzmalonsäuredi-2-ethylhexyl-ester;
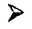 Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB);
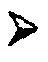 Propan-1,3-dione, wie z.B. 1-(4-tert.Butylphenyl)-3-(4'methoxyphenyl)propan-1,3-dion;
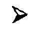 Ketotricyclo(5.2.1.0)decan-Derivate.

Als wasserlösliche Substanzen kommen in Frage:
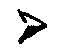 2-Phenylbenzimidazol-5-sulfonsäure und deren Alkali-, Erdalkali-, Ammonium-, Alkylammonium-, Alkanolammonium- und Glucammoniumsalze;
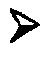 Sulfonsäurederivate von Benzophenonen, vorzugsweise 2-Hydroxy-4-methoxybenzo-phenon-5-sulfonsäure und ihre Salze;
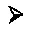 Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

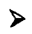 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

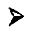 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

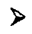 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

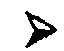 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
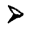 adstringierende Wirkstoffe,
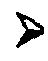 Ölkomponenten,
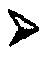 nichtionische Emulgatoren,
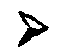 Coemulgatoren,
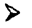 Konsistenzgeber,
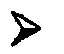 Hilfsstoffe wie z. B. Verdicker oder Komplexierungsmittel und/oder
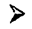 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
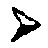 entzündungshemmende, hautschützende oder wohlriechende ätherische Öle,
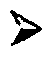 synthetische hautschützende Wirkstoffe und/oder
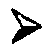 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können einschlägigen Werken entnommen werden

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
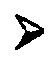 Glycerin;
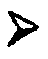 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
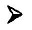 technische Oligoglyceringemische mit einem Eigenkondensationsgrad von 1,5 bis 10 wie etwa technische Diglyceringemische mit einem Diglyceringehalt von 40 bis 50 Gew.-%;
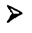 Methyolverbindungen, wie insbesondere Trimethylolethan, Trimethylolpropan, Trimethylolbutan, Pentaerythrit und Dipentaerythrit;
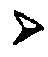 Niedrigalkylglucoside, insbesondere solche mit 1 bis 8 Kohlenstoffen im Alkylrest, wie beispielsweise Methyl- und Butylglucosid;
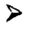 Zuckeralkohole mit 5 bis 12 Kohlenstoffatomen, wie beispielsweise Sorbit oder Mannit,
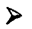 Zucker mit 5 bis 12 Kohlenstoffatomen, wie beispielsweise Glucose oder Saccharose;
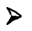 Aminozucker, wie beispielsweise Glucamin;
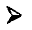 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung-Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Erfindungsgemäße Beispiele und Formulierungen

### Emulgator-Konzentrat 1 (erfindungsgemäß):

| | |
|---|---|
| Iso-Oleylglucosid | 50.9 Gew.-% |
| Iso-Oleylalkohol | 49,1 Gew.-% |
| Konsistenz | noch bei 5 °C flüssig; Viskosität von ca 500 mPa·s (Brookfield; RVS-Viskosimeter; 20°C, 10 UpM, Spindel 4, ohne Helipath) |

### Emulgator-Konzentrat 2 (Vergleichsbeispiel):

| | |
|---|---|
| Cetearylglucosid | 50.9 Gew.-% |
| Cetearylalkohol | 49,1 Gew.-% |
| Konsistenz | bei 25 °C fest, erst bei > 60 °C flüssig, nicht kaltverarbeitbar. |

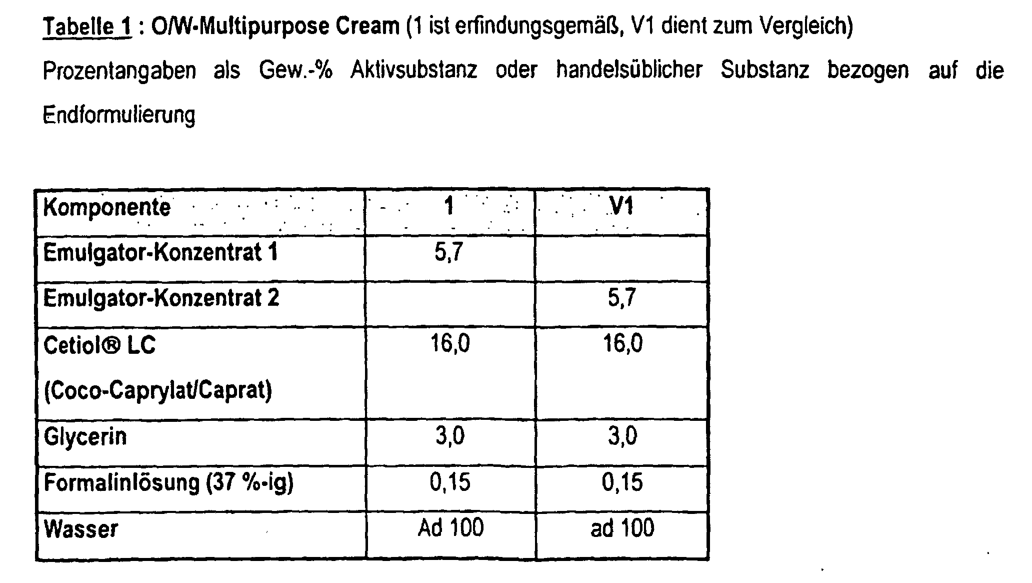

### Herstellung von Emulsionen gemäß Kaltverarbeitung

Es wurde eine wäßrige O/W-Emulsionen hergestellt unter Verwendung der in Tabelle 1 genannten Komponenten in der Weise, daß man unter Rühren bei 25 °C eine Mischung des Emulgator-Konzentrates 1 und Coco-Caprylate/- Caprate (Cetiol® LC, Fa. Cognis BV) als Ölphase in eine Mischung aus Glycerin, Formalinlösung und Wasser einrührte. Man erhielt eine über mehrere Monate auch unter Temperaturbelastung, stabile feinteilige Emulsion mit einer Viskosität von 24.800 mPa·s. Die Viskosität der Emulsion wurde nach der Brookfield-Methode in einem RVS-Viskosimeter (20°C, 10 UpM, Spindel 5, mit Helipath) bestimmt.

### Herstellung von Emulsionen gemäß Heißverarbeitung (V1)

Das Vergleichsbeispiel V1 kann ausschließlich gemäß Heißverarbeitung hergestellt werden. Hierzu wurde eine Mischung des Emulgator-Konzentrates 2 und Coco-Caprylate/-Caprate (Cetiol® LC, Fa. Cognis BV) auf 80 °C erhitzte und dann in eine auf 80 °C erwärmte Mischung aus Glycerin und Wasser eingerührt. Nach Abkühlen auf Raumtemperatur wurde die Emulsion mit Formalinlösung konserviert. Man erhielt eine Emulsion, die bezüglich Stabilität der erfindungsgemäßen Zubereitung unterlegen ist.

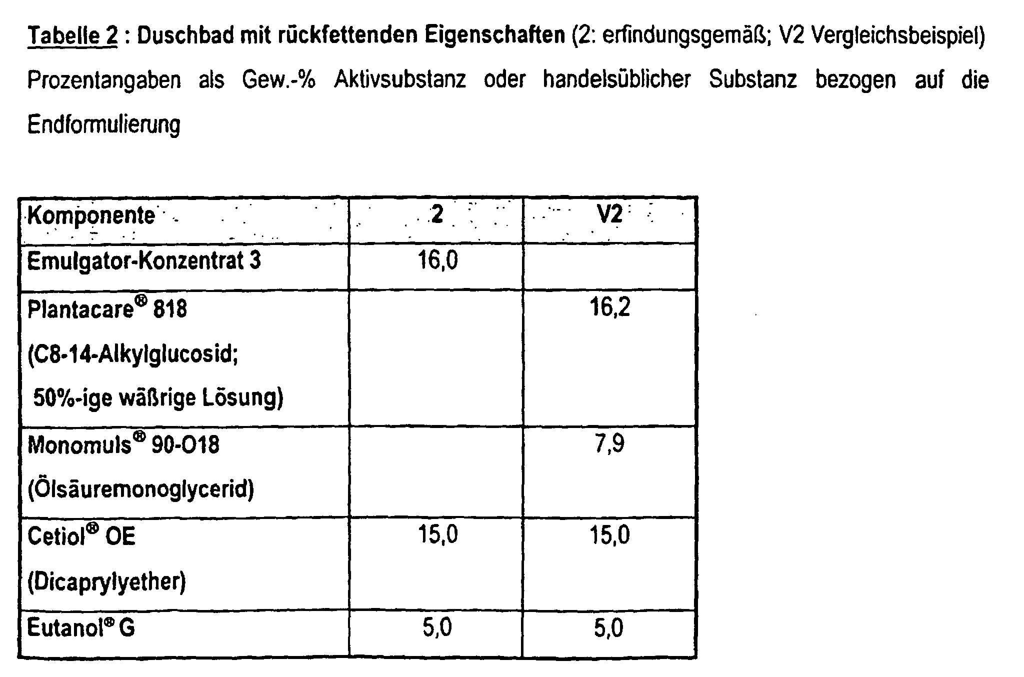

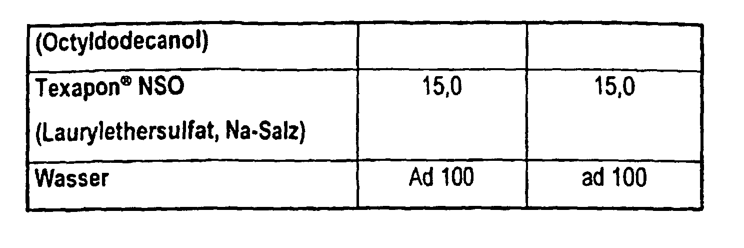

### Emulgator-Konzentrat 3 (erfindungsgemäß):

| | |
|---|---|
| Iso-Oleylglucosid | 50.6 Gew.-% |
| Iso-Oleylalkohol | 49,4 Gew.-% |

Die Schaumbadformulierung 2 (Tabelle 2) wurde in der Weise hergestellt, daß man unter Rühren bei 25 °C das Konzentrat 3, Dicaprylylether (Cetioi®OE, Fa. Cognis BV) und 2-Octyldodecanol (Eutanol®G) in eine Mischung aus Fettalkoholethersulfat (Texapon®NSO) und Wasser einrührte. Man erhielt ein flüssiges milchiges Konzentrat.

Die Schaumbadformulierung V2 (Tabelle 2) wurde in der Weise hergestellt, daß man unter Rühren bei 25 °C eine Mischung aus Ölsäuremonoglycerid (Monomuls®90-018, Fa. Cognis BV), Dicaprylylether (Cetiol®OE, Fa. Cognis BV) und 2-Octyldodecanol (Eutanol®G) in eine Mischung aus einer 50 %-igen wäßrigen C8-14-Alkylglucosid-Lösung (Plantacare®818, Fa. Cognis BV), Fettalkoholethersulfat (Texapon®NSO) und Wasser einrührte. Man erhielt ein flüssiges milchiges Konzentrat.

Aufgrund seiner bei Raumtemperatur festen Konsistenz läßt sich Emulgatorkonzentrat 2 nicht in Shampoo- oder Duschbadformulierung einarbeiten, da es zur Auskristallisation kommt. Prinzipiell sind als Rückfetter für Duschbadformulierungen Substanzen geeignet, die bei 20 °C flüssig sind.

### Anwendungstechnische Bewertung

Zur Beurteilung der dermatologischen Veträglichkeit wurde der transepidermale Wasserverlust an einer Schweineepidermis untersucht. Hierzu wurden definierte Hautstücke bei 40°C über einen Zeitraum von 30 min mit je einer 20 %-igen wäßrigen Lösung der nach Beispiel 2 (erfindungsgemäß) und Beispiel V2 (=Vergleichsbeispiel) hergestellten Schaumbad-Rezepturen behandelt und der TEWL-Wert gravimetrisch bestimmt.

Die Ergebnisse sind in Tabelle 3 zusammengefaßt. Als TEWL-Wert wird hierbei das in Prozent ausgedrückte Verhältnis des transepidermalen Wasserverlustes einer unbehandelten zu einer behandelten Probe angegeben. Je niedriger der Wert ist, um so besser ist die dermatologische Verträglichkeit.

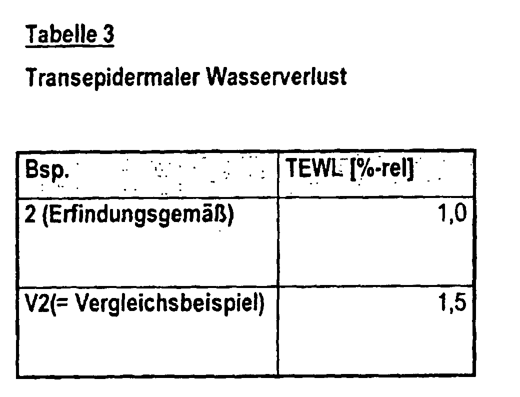

Die erfindungsgemäße Zubereitung zeigt einen deutlich geringeren transepidermalen Wasserverlust als die Vergleichsformulierung, bei der Ölsäuremonoglycerid als typischer Rückfetter eingesetzt wurde. Für die erfindungsgemäße Formulierung ergibt sich somit eine signifikant bessere dermatologische Verträglichkeit.

## Patentansprüche

1. Emulgator-Konzentrat enthaltend
(a) 0,5 bis 90 Gew.-% wenigstens eines iso-Alkenyloligoglykosids,
(b) 0,5 bis 90 Gew.-% wenigstens eines iso-Alkenyl-Fettalkohols und
(c) 0 bis 10 Gew.-% Wasser.

2. Emulgator-Konzentrat gemäß Anspruch 1, **dadurch gekennzeichnet daß** es
(a) 15 bis 80 Gew.-% wenigstens eines iso-Alkenyloligoglykosids,
(b) 20 bis 85 Gew.-% wenigstens eines iso-Alkenyl-Fettalkohols und
(c) 0 bis 10 Gew.-% Wasser enthält.

3. Emulgator-Konzentrat gemäß einem der Ansprüche 1 bis 2, **dadurch gekennzeichnet, daß** der iso-Alkenylrest des Fettalkohols und des Oligoglykosids identisch sind.

4. Emulgator-Konzentrat gemäß einem der Ansprüche 1 bis 3, **dadurch gekennzeichnet, daß** sie iso-Alkenyloligoglykoside der Formel (I) enthalten,
R¹O-[G]ₚ (I)
in der R¹ für einen iso-Alkenylrest mit 10 bis 54, vorzugsweise mit 16 bis 22 Kohlenstoffatomen, G für einen Zuckerrest mit 5 oder 6 Kohlenstoffatomen und p für Zahlen von 1 bis 10 steht.

5. Emulgator-Konzentrat gemäß einem der Ansprüche 1 bis 4, **dadurch gekennzeichnet, daß** es
(a) 0,5 bis 90 Gew.-% wenigstens eines iso-Oleyl-Oligoglukosids,
(b) 0,5 bis 90 Gew.-% wenigstens eines iso-Oleylalkohols und
(c) 0- 10 Gew.-% Wasser
enthält.

6. Emulgator-Konzentrat gemäß einem der Ansprüche 1 bis 5, **dadurch gekennzeichnet, daß** es zusätzlich wenigstens ein hydrophiles Wachs enthält.

7. Kosmetische oder pharmazeutische Zubereitungen enthaltend 0,1 bis 30 Gew.-% der Emulgatorkonzentrate gemäß einem der Ansprüche 1 bis 5.

8. Verwendung eines Emulgator-Konzentrates gemäß einem der Ansprüche 1 bis 5 zur Herstellung kosmetischer oder pharmazeutischer Zubereitungen.

9. Verwendung eines Emulgator-Konzentrates gemäß einem der Ansprüche 1 bis 5 als Rückfettungsmittel in kosmetischen oder pharmazeutischen Zubereitungen.

10. Verfahren zur Herstellung von Emulgator-Konzentraten nach einem der Ansprüche 1 bis 5, bei dem man einen Zucker (Ose) und überschüssigen iso-Alkenylalkohol einer sauren Acetalisierung unterwirft
(a) wobei das Edukt-Verhältnis von Zucker zu iso-Alkenyl-Fettalkohol so eingestellt wird, daß hieraus nach der Synthese direkt das gewünschte Verhältnis von iso-Alkenyloligoglykosid zu iso-Alkenyl-Fettalkohol resultiert
oder in dem resultierenden Gemisch aus iso-Alkenyloligoglykosiden und überschüssigem iso-Alkenyl-Fettalkohol
(b) der iso-Alkenyl-Feftalkoholanteil im Gemisch entweder durch Abdestillieren des Fettalkohols oder durch Zugabe von zusätzlichem iso-Alkenyloligoglykosid-Anteil auf den gewünschten Gehalt reduziert wird oder
(c) der iso-Alkenyl-Fettalkoholanteil im Gemisch durch nachträgliche Zugabe von iso-Alkenyl-Fettalkohol auf den gewünschten Gehalt erhöht.

## Claims

1. An emulsifier concentrate containing:
(a) 0.5 to 90% by weight of at least one isoalkenyl oligoglycoside,
(b) 0.5 to 90% by weight of at least one isoalkenyl fatty alcohol and
(c) 0 to 10% by weight of water.

2. An emulsifier concentrate as claimed in claim 1, **characterized in that** it contains
(a) 15 to 80% by weight of at least one isoalkenyl oligoglycoside,
(b) 20 to 85% by weight of at least one isoalkenyl fatty alcohol and
(c) 0 to 10% by weight of water.

3. An emulsifier concentrate as claimed in claim 1 or 2, **characterized in that** the isoalkenyl groups of the fatty alcohol and the oligoglycoside are identical.

4. An emulsifier concentrate as claimed in any of claims 1 to 3, **characterized in that** it contains isoalkenyl oligoglycosides corresponding to formula **(I)**:
R¹O-[G]ₚ (I)
in which R¹ is an isoalkenyl group containing 10 to 54 and preferably 16 to 22 carbon atoms, G is a sugar unit containing 5 or 6 carbon atoms and p is a number of 1 to 10.

5. An emulsifier concentrate as claimed in any of claims 1 to 4, **characterized in that** it contains
(a) 0.5 to 90% by weight of at least one iso-oleyl oligoglucoside,
(b) 0.5 to 90% by weight of at least one iso-oleyl alcohol and
(c) 0 to 10% by weight of water.

6. An emulsifier concentrate as claimed in any of claims 1 to 5, **characterized in that** it additionally contains at least one hydrophilic wax.

7. Cosmetic or pharmaceutical preparations containing 0.1 to 30% by weight of the emulsifier concentrates as claimed in any of claims 1 to 5.

8. The use of the emulsifier concentrate claimed in any of claims 1 to 5 for the production of cosmetic or pharmaceutical preparations.

9. The use of an emulsifier concentrate as claimed in any of claims 1 to 5 as a lipid layer enhancer in cosmetic or pharmaceutical preparations.

10. A process for the production of the emulsifier concentrates claimed in any of claims 1 to 5, **characterized in that** a sugar (ose) and excess isoalkenyl fatty alcohol are subjected to acidic acetalization,
(a) the educt ratio of sugar to isoalkenyl fatty alcohol being adjusted so that the required ratio of isoalkenyl oligoglycoside to isoalkenyl fatty alcohol is directly achieved after the synthesis
or, in the resulting mixture of isoalkenyl oligoglycosides and excess isoalkenyl fatty alcohol,
(b) the isoalkenyl fatty alcohol content of the mixture being reduced to the required level either by distilling off the fatty alcohol or by addition of more isoalkenyl oligoglycoside or
(c) the isoalkenyl fatty alcohol content of the mixture being increased to the required level by subsequent addition of isoalkenyl fatty alcohol.

## Revendications

1. Concentré d'émulsifiant contenant
(a) 0,5 à 90 % en poids d'au moins un iso-alcényloligoglycoside,
(b) 0,5 à 90 % en poids d'au moins un alcool gras iso-alcénylique et
(c) 0 à 10 % en poids d'eau.

2. Concentré d'émulsifiant selon la revendication 1,
**caractérisé en ce qu'**
il contient
(a) 15 à 80 % en poids d'au moins un iso-alcényloligoglycoside,
(b) 20 à 85 % en poids d'au moins un alcool gras iso-alcénylique et
(c) 0 à 10 % en poids d'eau.

3. Concentré d'émulsifiant selon l'une des revendications 1 à 2,
**caractérisé en ce que**
les radicaux iso-alcényle de l'alcool gras et de l'oligoglycoside sont identiques.

4. Concentrés d'émulsifiants selon l'une des revendications 1 à 3,
**caractérisés en ce qu'**
ils contiennent des iso-alcényloligoglycosides de formule (I)
R¹-O-[G]ₚ (I)
dans laquelle R¹ représente un radical iso-alcényle comportant de 10 à 54, de préférence de 16 à 22 atomes de carbone, G représente un radical saccharique comportant 5 ou 6 atomes de carbone, et p représente des nombres allant de 1 à 10.

5. Concentré d'émulsifiant selon l'une des revendications 1 à 4,
**caractérisé en ce qu'**
il contient
(a) 0,5 à 90 % en poids d'au moins un iso-oléyl-oligoglucoside,
(b) 0,5 à 90 % en poids d'au moins un alcool iso-oléique et
(c) 0 à 10 % en poids d'eau.

6. Concentré d'émulsifiant selon l'une des revendications 1 à 5,
**caractérisé en ce qu'**
il contient en outre au moins une cire hydrophile.

7. Préparations cosmétiques ou pharmaceutiques contenant de 0,1 à 30 % en poids de concentrés d'émulsifiants selon l'une des revendications 1 à 5.

8. Utilisation d'un concentré d'émulsifiant selon l'une des revendications 1 à 5, pour la production de préparations cosmétiques ou pharmaceutiques.

9. Utilisation d'un concentré d'émulsifiant selon l'une des revendications 1 à 5, comme agent de regraissage dans des préparations cosmétiques ou pharmaceutiques.

10. Procédé de production de concentrés d'émulsifiants selon l'une des revendications 1 à 5, dans lequel on soumet un sucre (ose) et un excès d'alcool iso-alcénylique à une acétalisation acide, selon lequel
(a) on règle le rapport du sucre et de l'alcool iso-alcénylique de façon à obtenir directement, après la synthèse, le rapport désiré de l'iso-alcényloligoglycoside et de l'alcool gras iso-alcénylique,
ou dans le mélange résultant d'iso-alcényloligoglycosides et d'alcool gras isoalcénylique en excès
(b) on réduit la proportion d'alcool gras iso-alcénylique à la valeur désirée, soit par distillation de cet alcool, soit en ajoutant une fraction supplémentaire d'iso-alcényloligoglycoside, ou
(c) on élève à la valeur désirée la proportion d'alcool gras iso-alcénylique en effectuant une addition ultérieure de cet alcool.
